# EUROPEAN PATENT APPLICATION

(11) **EP 1 375 476 A1**
(43) Date of publication of application: **02.01.2004**
(21) Application number: 02013950.7
(22) Date of filing: 25.06.2002
(51) Int. Cl.: C07C 291/02, C07C 239/20, C07D 211/94, C07B 61/02, C08F 4/00

(54) **A new process for the synthesis of alkoxyamines active in controlled radical polymerization**

(71) Applicant: Bayer Aktiengesellschaft, 51368 Leverkusen (DE)
(72) Inventor: Detrembleur, Christophe, Dr., 51061 Köln (DE); Gross, Thomas, Dr., 42489 Wülfrath (DE); Meyer, Rolf-Volker, Dr., 53804 Much (DE)

(57) **Abstract**

The present invention relates to a new process for the preparation of alkoxyamine initiators and to a process of radical polymerization using the alkoxyamine initiators as intermediates.

## Description

The present invention relates to a new process for the preparation of alkoxyamine initiators and to a process of radical polymerization using the alkoxyamine initiators as intermediate.

The use of the controlled radical polymerization ("CRP") of vinyl monomers has increased rapidly because it allows the synthesis of a broad range of well-defined (co)polymers under uncomplicated experimental conditions. The polymerization can, for example, be carried out in aqueous media and under moderate polymerization temperatures and purification of the monomer prior to polymerization is not required. Additionally, the main molecular parameters of the polymer chain, for example its polydispersity, molecular weight, polymer architecture or the structure of the chain-ends can be easily controlled and adjusted. The CRP is also called "living" free radical polymerization. The aim of the precise control of free radical polymerization is achieved by reversible chain termination or blocking ("end-capping") after each growth step. The equilibrium concentration of the polymerization-active ("living") chain ends in this case is so low compared with the equilibrium concentration of the blocked ("dormant") chain ends that irreversible termination and transfer reactions are greatly suppressed compared with the growth reaction. Since the end-capping proceeds reversibly, all the chain ends remain "living" if no termination reagent is present. This allows the control of the molecular weight, low polydispersity and controlled functionalization of the chain ends by termination reagents.

Of all the CRP systems presently under investigation, the nitroxyl-mediated polymerization ("NMP") is one of the most attractive and efficient, because this technique provides advantages applicable to a broad range of monomers such as (meth)acrylates, acrylonitrile, styrenes, acrylamides, butadiene or isoprene and can be carried out in a metal-free, colorless and odorless manner.

Numerous publications have shown that alkoxyamines can be used to initiate and control the radical polymerization of vinyl monomers according to an NMP mechanism.

US-A 4,581,429 discloses alkoxyamines which are formed by the reaction of linear or cyclic nitroxides, such as 2,2,6,6-tetramethylpiperidin-1-oxyl (TEMPO) with organic carbon-based free radicals, and a process for the preparation of vinyl polymers using these compounds as initiators. The reactions typically have a low concentration of free radicals which, in the free radical polymerization of vinyl monomers, means that bimolecular termination reactions are less likely to occur than unimolecular growth reactions.

Other examples are described by *Hawker et al. (J. Am. Chem. Soc.* **1994**, 116, 11185 and J *Am. Chem. Soc.* **1999**, 121, 3904-3920) and in US-A 5,322,912, US-A 5,412,047, US-A 5,449,724, US-A 5,498,679, US-A 6,258,911, DE-A 199 09 767 and EP-A 0 891 986.

The most commonly used method for the synthesis of alkoxyamines consists in coupling an alkyl radical to a nitroxyl radical. The alkyl radical can be generated by different methods, for example by decomposition of azo compounds (*Hawker et al.,* Macromolecules 1996, 29, 5245-5254; *Yozu Miura et al.,* Macromolecules 1998, 31, 6727-6729), by hydrogen removal from an appropriate substrate (*Hawker et al.,* Macromolecules 1996, 29, 5245-5254; *Yozu Miura et al.,* Macromolecules 1998, 31, 4659-4661) or by addition of a radical to an olefin (*Hawker et al., J.* Am. Chem. Soc. 1994, 116, 11185). The alkyl radical can also be generated from an halogenated compound R-X in the presence of a metallic catalyst following an atom transfer radical addition ("ATRA") reaction (WO-A 00/49027; WO-A 00/61544).

EP-A 1 083 169 discloses a process for the preparation of functional alkoxyamine initiators in which hydrogen peroxide is reacted with iron(II) sulfate in the presence of a nitroxyl radical and a vinyl monomer to form the alkoxyamine with a good yield in a one-pot process.

The major disadvantage of the methods described above is that the alkoxyamines have to be synthesised from costly nitroxyl radicals and generally must be purified before they can be used for polymerization.

The object of the present invention was to provide a new synthetic pathway for the synthesis of alkoxyamines in a one-pot process starting from hindered secondary amines and to use these alkoxyamines as intermediates in a polymerization process which provides homo- and copolymers of narrow polydispersity with a specific molecular weight and which does not have the above-mentioned disadvantages of the prior art.

Surprisingly, it has now been found that alkoxyamines can be produced from secondary amines in a one-pot process and used, without intermediate purification, in a controlled free-radical polymerization process.

The object of the present invention is a one-pot process for the preparation of alkoxyamines of the general formula (I) or (II) 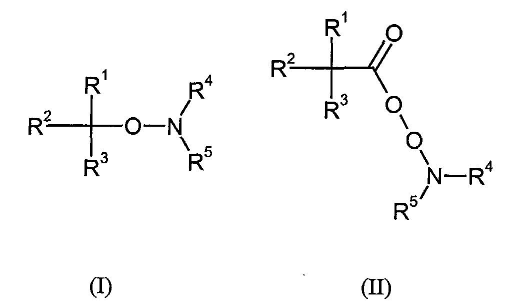 wherein
- R¹, R², R³: are independently selected from the group consisting of: hydrogen, C₁-C₂₀alkyl, C₁-C₂₀cycloalkyl, C₆-C₂₄aryl, halogen, cyano, hydroxy, amino, C₁-C₂₀alkylester C₁-C₂₀cycloalkylester, C₁-C₂₀alkylamide, C₁-C₂₀cycloalkylamide C₆-C₂₄-arylester or C₆-C₂₄-arylamide; and
- R¹, R², R³: optionally form, together with the intermediate carbon atom, a C₃-C₁₂cycloalkyl radical, a (C₄-C₁₂alkanol)yl radical or a C₂-C₁₃-heterocycloalkyl radical containing oxygen, sulfur or nitrogen atoms;
wherein optionally at least one of the radicals R¹, R² and R³ contains a functional group Y which is capable of further reacting or crosslinking with the functional groups known from the coatings field;
- R⁴ and R⁵: are independently selected from the group consisting of: C₁-C₁₈alkyl, C₂-C₁₈alkenyl, C₂-C₁₈alkynyl, C₃-C₁₂cycloalkyl or C₃-C₁₂heterocycloalkyl, C₆-C₂₄-aryl, which are unsubstituted or substituted by NO₂, halogen, amino, hydroxy, cyano, carboxy, ketone, C₁-C₄alkoxy, C₁ -C₄alkylthio, C₁-C₄alkylamino, dialkyl phosphonates;
- R⁴ and R⁵: optionally form, together with the intermediate nitrogen atom, a C₃-C₁₂cycloalkyl radical, a (C₄-C₁₂alkanol)yl radical or a C₂-C₁₃-heterocycloalkyl radical containing oxygen, sulfur or nitrogen atoms;
- R⁴ and R⁵: together form a residue of a polycyclic ring system or a polycyclic heterocycloaliphatic ring system containing oxygen, sulfur or nitrogen atoms;
wherein the carbon atom of the R⁴ and R⁵ radicals directly adjacent to the alkoxyamine nitrogen atom is in each case substituted by 3 further organic substituents;
wherein optionally at least one of the radicals R⁴ and R⁵ contains a functional group Y which is capable of further reacting or crosslinking with the functional groups known from the coatings field;
comprising the reaction steps of
(1) reacting of an oxidizing agent (A) with a sterically hindered secondary amine of the general formula (III), 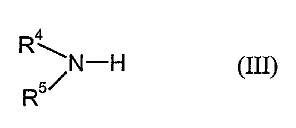 wherein
   - R⁴ and R⁵: are independently selected from the group consisting of: C₁-C₁₈alkyl, C₂-C₁₈alkenyl, C₂-C₁₈alkynyl, C₃-C₁₂cycloalkyl or C₃-C₁₂heterocycloalkyl, C₆-C₂₄aryl, which are unsubstituted or substituted by NO₂, halogen, amino, hydroxy, cyano, carboxy, ketone, C₁-C₄alkoxy, C₁-C₄alkylthio, C₁-C₄alkylamino, dialkyl phosphonates;
   - R⁴ and R⁵: optionally form, together with the intermediate nitrogen atom, a C₃-C₁₂cycloalkyl radical, a (C₄-C₁₂alkanol)yl radical or a C₂-C₁₃heterocycloalkyl radical containing oxygen, sulfur or nitrogen atoms;
   - R⁴ and R⁵: together form a residue of a polycyclic ring system or a polycyclic heterocycloaliphatic ring system containing oxygen, sulfur or nitrogen atoms;
   wherein the carbon atom of the R⁴ and R⁵ radicals directly adjacent to the alkoxyamine nitrogen atom is in each case substituted by 3 further organic substituents;
   wherein optionally at least one of the radicals R⁴ and R⁵ contains a functional group Y which is capable of further reacting or crosslinking with functional groups known in the coatings field;
   in a water-containing medium,
(2) removing of the aqueous phase,
(3) adding a free-radical initiator (B) and starting the decomposition to generate radicals of the general formulae (IV) or (V): 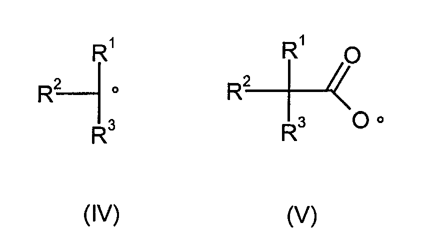
wherein
- R¹, R², R³: are independently selected from the group consisting of: hydrogen, hydroxy, cyano, C₁-C₂₀alkyl, C₁-C₂₀cycloalkyl, C₆-C₂₄aryl, halogen, cyano, hydroxy, amino, C₁-C₂₀alkylester C₁-C₂₀cycloalkylester, C₁-C₂₀alkylamide, C₁-C₂₀cycloalkylamide C₆-C₂₄-arylester or C₆-C₂₄-arylamide;
- R¹, R², R³: optionally form, together with the intermediate carbon atom, a C₃-C₁₂cycloalkyl radical, a (C₄-C₁₂alkanol)yl radical, a phenyl radical or a C₂-C₁₃-heterocycloalkyl radical containing oxygen, sulfur or nitrogen atoms;
wherein optionally at least one of the radicals R¹, R² and R³ contains a functional group Y which is capable of further reacting or crosslinking with the functional groups known from the coatings field.

The Y group is capable of reacting further or crosslinking and is for example hydroxyl, carboxy, amino, isocyanate, urethane or epoxide groups.

Suitable oxidizing agents (A) can be all oxidizing agents known from the prior art for the oxidation of secondary amines into nitroxyl radicals. Preferred oxidizing agents are water-soluble oxidizing agents, such as peracids such as peracetic acid, perpropionic acid, m-chloroperbenzoic acid, dimethyldioxirane, perbenzoic acid, or peroxides such as potassium peroxymonosulfate (Oxone®, DuPont Specialty Chemistry, USA), hydrogen peroxide, hydrogen peroxide/sodium tungstate, hydrogen peroxides/titanium containing catalysts, such as for example titanium dioxide and titanium silicalites (EP-A 0 488 403, page 5), phosphotungstic acid and oxidizing gases such as molecular oxygen or ozone. Particularly preferred are peracetic acid, perpropionic acid, m-chloroperbenzoic acid, Oxone® (DuPont Specialty Chemistry, USA) and hydrogen peroxide/sodium tungstate.

Metal oxides such as silver oxide, lead (IV) oxide and sodium tungstate can also be used, optionally in combination with another oxidizing agent. A mixture of various oxidizing agents can also be used.

Suitable free-radical initiators (B) of the present invention are any suitable agents producing free-radicals able to react with nitroxyl radicals, for example precursors such as azo compounds, and some peroxides, which generate radicals by thermolysis or precursors such as styrene, which generate radicals by autopolymerization. It is also possible to generate radicals by redox systems, photochemical systems or by high energy radiation such as beam or X- or γ- radiation.

Other useful systems to generate radicals are organometallic compounds such as Grignard reagents (Hawker et al., Macromolecules 1996, 29, 5245) or halogenated compounds which are produced in the presence of a metal complex according to an Atom Transfer Radical Addition Process (ATRA) (WO-A 00/61544).

Examples of free radical initiators (B) generating free-radicals by thermolysis are 2,2'-azobis(isobutyronitrile) (AiBN), 2,2'-azobis(isovaleronitrile), 2,2'-azobis(methylisobutyrate), 4,4'-azobis(4-cyanopentanoicacid), 1,1'-azobis(1 -cyclohexanecarbonitrile), 2-tert-butylazo-2-cyanopropane, 2,2'-azobis[2-methyl-N-(1,1-bis(hydroxymethyl)-2-hydroxyethylpropionamide], 2,2'-azobis[2-methyl-N-(2-hydroxyethyl) propionamide], 2,2'-azobis(isobutyramidinehydrochloride), 2,2'-azobis(N,N'-dimethyleneisobutyramine), 2,2'-azobis[2-methyl-N-(1,1-bis(hydroxymethyl)-2-ethyl)-propionamide], 2,2'-azobis[2-methyl-N-(2-hydroxyethylpropionamide], 2,2'-azobis(isobutyamide)dihydrate, 2,2'-azobis(2,2,4-trimethylpentane), 2,2'-azobis(2-methylpropane), and dibenzoylperoxide.

Initiators generating radicals by photolysis are for example benzoin derivatives, benzophenone, acyl phosphine oxides and photoredox systems.

Initiators generating radicals as a result of a redox reaction are in general a combination of an oxidant and a reducing agent. A suitable oxidant is for example, benzoylperoxide. Suitable reducing agents are for example Fe(II) salts, Ti(III) salts, potassium thiosulfate, potassium bisulfite, ascorbic acid and its salts, oxalic acid and salts, dextrose and Rongalite® (sodium formaldehyde sulfoxylate, BASF AG, Ludwigshafen, DE).

Preferred radical initiator (B) are compounds which generate free-radicals by thermolysis, particularly preferred in AIBN and dibenzoylperoxide.

The water phase in which the secondary amine is dispersed can contain a basic organic or inorganic buffer or organic or inorganic bases, such as Na₂CO₃, NaHCO₃, K₂CO₃, KHCO₃, Na₃PO₄, Na₂HPO₄, NaH₂PO₄, metal salts of carboxylic acids such as acetic acid sodium salt or propionic acid sodium salt, or a mixture thereof. Na₂CO₃, NaHCO₃, K₂CO₃, KHCO₃ and the sodium, calcium or potassium salts of acetic acid are preferred.

Useful sterically hindered secondary amines of the general formula (III) are for example those of the following formulae (VI) to (X): 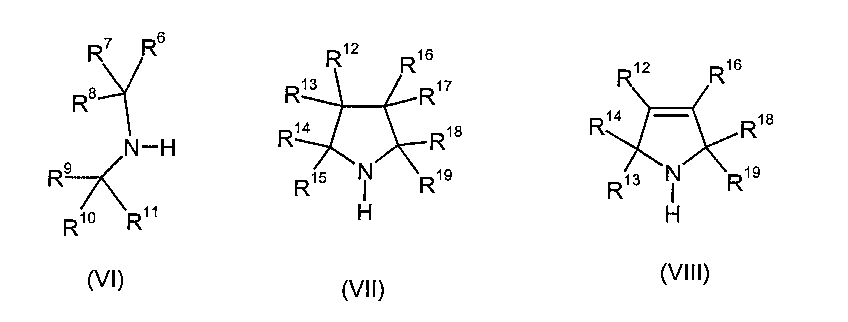 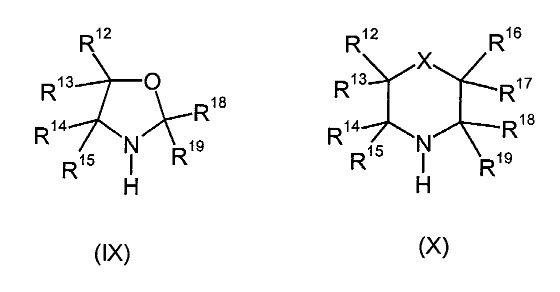 wherein
- R⁶, R⁷, R⁸,R⁹,R¹⁰,^{R11},R¹²,R¹³, R¹⁴,R¹⁵,R¹⁶, R¹⁷, R¹⁸, R¹⁹: are independently selected from the group consisting of: hydrogen, halogen or cyano-, amide-, ether-, ester-, thioether-, ketone-, amide-, carboxyl-, amidine- or dialkylphosphonyl-containing groups;
- R⁶ to R¹⁹: are independently selected form the group consisting of: C₁-C₁₈alkyl, C₂-C₁₈alkenyl, C₂-C₁₈alkynyl, C₃-C₁₂cycloalkyl or C₃-C₁₂heterocycloalkyl, C₆-C₂₄aryl, which are unsubstituted or substituted by NO₂, halogen, amino, hydroxy, cyano, carboxy, C₁-C₄alkoxy, C₁-C₄alkylthio, C₁-C₄alkylamino;
- R⁶ to R¹⁹: can form, together with the intermediate carbon atom, a C₃-C₁₂-cycloalkyl radical, a (C₄-C₁₂alkanol)yl radical or a C₂-C₁₃heterocycloalkyl radical containing oxygen, sulfur or nitrogen atoms;
- R⁶ to R¹⁹: together form a residue of a polycyclic ring system or a polycyclic heterocycloaliphatic ring system containing oxygen, sulfur or nitrogen atoms;
wherein optionally at least one of the radicals R⁶ to R¹⁹ contains a functional group Y which is capable of reacting further or of cross-linking with functional groups known in the coatings field and
- X: represents a methylene, ketone, ester group or oxygen atom, a hydrocarbon radical, which can be substituted by a cyano, ester, hydroxy, nitro, ether or imido group.

Other useful secondary amines are for example those of the following formulare (XI) and (XII): 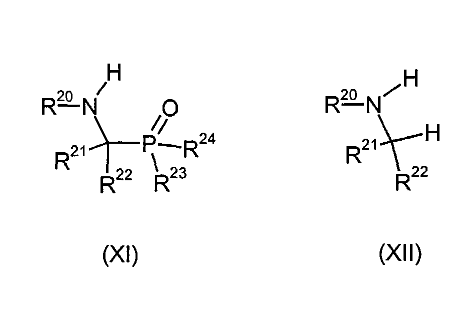 wherein
- R²⁰: is selected from the group consisting of: C₁-C₁₈alkyl, C₂-C₁₈alkenyl, C₂-C₁₈alkynyl, C₃-C₁₂cycloalkyl or C₃-C₁₂heterocycloalkyl, C₆-C₂₄-aryl, which are unsubstituted or substituted by NO₂, halogen, amino, hydroxy, cyano, carboxy, C₁-C₄alkoxy, C₁-C₄alkylthio, C₁-C₄alkylamino;
- R²⁰: optionally contain a functional group Y which is capable of reacting further or of crosslinking with the functional groups known from the coatings field;
- R²¹, R²²: are independently selected from the group consisting of: hydrogen, halogen or cyano-, amide-, ether-, ester-, thioether-, ketone-, amide-, carbomyl-, amidine- or dialkylphosphonyl-containing groups;
- R²¹, R²²: are independently selected from the group consisting of: C₁-C₁₈alkyl, C₂-C₁₈alkenyl, C₂-C₁₈alkynyl, C₃-C₁₂cycloalkyl or C₃-C₁₂heterocycloalkyl, C₆-C₂₄aryl, which are unsubstituted or substituted by NO₂, halogen, amino, hydroxy, cyano, carboxy, C₁-C₄alkoxy, C₁-C₄-alkylthio or C₁-C₄alkylamino;
wherein R²¹ and R²² optionally form, together with the intermediate carbon atom a C₃-C₁₂cycloalkyl radical, a (C₄-C₁₂alkanol)yl radical or a C₂-C₁₃heterocycloalkyl radical containing oxygen, sulfur or nitrogen atoms;
- R²¹, R²²: together form a residue of a polycyclic ring system or a polycyclic heterocycloaliphatic ring system containing oxygen, sulfur or nitrogen atoms;
wherein R²³ and R²⁴ optionally form, together with the intermediate phosphorus atom a C₃-C₁₂cycloalkyl radical, a (C₄-C₁₂alkanol)yl radical or a C₂-C₁₃heterocycloalkyl radical containing oxygen, sulfur or nitrogen atoms;
wherein optionally at least one of the radicals R²⁰ to R²⁴ contains a functional group Y which is capable of further reacting or of crosslinking with functional groups known from the coatings field;
- R²³, R²⁴: are independently selected from the group consisting of: C₁-C₁₈alkyl, C₂-C₁₈alkenyl, C₂-C₁₈alkynyl, C₃-C₁₂cycloalkyl or C₃-C₁₂heterocycloalkyl or C₆-C₂₄aryl, which are unsubstituted or substituted by NO₂, halogen, amino, hydroxy, cyano, carboxy, C₁-C₄alkoxy, C₁-C₄alkylthio or C₁-C₄alkylamino;
wherein R²³ and R²⁴ optionally form, together with the intermediate phosphorus atom, a C₃-C₁₂cycloalkyl radical, a (C₄-C₁₂alkanol)yl radical or a C₂-C₁₃heterocycloalkyl radical containing oxygen, sulfur or nitrogen atoms;
wherein at least one of the radicals R²³ to R²⁴ optionally contains a functional group Y which is capable of further reacting or of cross-linking with functional groups is known from the coatings field.

Preferred secondary amines of the general formula (III) are tert-butyl amine; 2,2,6,6-tetramethylpiperidine; 4-hydroxy-2,2,6,6-tetramethylpiperidine; 2,2,6,6-tetramethyl-4-piperidinone; 2,2,6,6-tetramethyl-4-piperidinyl acetate; 2,2,6,6-tetramethyl-4-piperidinyl stearate; 2,2,6,6-tetramethyl-4-piperidinyl benzoate; 2,6-dimethyl-2,6-diethylpiperidine; diethyl 1-(tert-butylamino)-2,2-dimethylpropylphosphonate; dipropyl 1-(tert-butylamino)-2,2-dimethylpropylphosphonate; dibutyl 1-(tert-butylamino)-2,2-dimethylpropylphosphonate; N-(tert-butyl)-1-(diethylphosphoryl)-2,2-dimethyl-1 -propylamine; N-(tert-butyl)-1-(dipropylphosphoryl)-2,2-dimethyl-1-propylamine; N-(tert-butyl)-2-methyl-1-phenyl-1-propylamine; 2,2,4,6,6-pentamethyl-1,2,5,6-tetrahydropyrimidine; N-[(3E)-2,2-diphenyl-1,2-dihydro-3H-indol-3-yl-idene]-N-phenylamine; 2,6-diethyl-2,3,6-trimethyl-4-piperidinone; 2,6-diethyl-2,3,6-trimethyl-4-piperidinol; 14-oxa-7-azadispiro[5.1.5.2]pentadecane; 2,2,4,4-tetramethyl-1,3-oxazolidine; 2,2,5,5-tetramethyl-1-pyrrolidine; 3-carboxy-2,2,5,5-tetramethyl-1-pyrrolidine; 2,5-diphenyl-2,5-dimethylpyrrolidine; 3-carboxy-2,5-diphenyl-2,5-dimethylpyrrolidine; 1,1,3,3-tetraethylisoindoline; 1,1,3,3-tetramethylisoindoline; 1,1,3,3-tetrapropylisoindoline.

Particularly preferred are: tert-butyl amine; 2,2,6,6-tetramethylpiperidine; 4-hydroxy-2,2,6,6-tetramethylpiperidine; 2,2,6,6-tetramethyl-4-piperidinone; 2,2,6,6-tetramethyl-4-piperidinyl acetate; diethyl 1-(tert-butylamino)-2,2-dimethylpropyl phosphonate; dipropyl 1-(tert-butylamino)-2,2-dimethylpropyl phosphonate; dibutyl 1-(tert-butylamino)-2,2-dimethylpropyl phosphonate; 2,6-diethyl-2,3,6-trimethyl-4-piperidinone; 2,6-diethyl-2,3,6-trimethyl-4-piperidinol; 2,2,5,5-tetramethyl-1-pyrrolidine; 1,1,3,3-tetramethylisoindoline.

Polyfunctional amines can also be used as compounds of the formula (III) in order to form resins displaying heat reversibility. In the context of the present invention polyfunctional amines are compounds which have more than one secondary amino group. These properties are particularly interesting when low viscosity of the polymer is required during processing.

Some examples of suitable polyfunctional amines are bis(2,2,6,6-tetramethylpiperidine) sebacate; bis(2,2,6,6-tetramethylpiperidine) succinate; bis(2,2,6,6-tetramethylpiperidine) adipate; bis(2,2,6,6-tetramethylpiperidine) phthalate; bis(2,2,6,6-tetramethylpiperidine) isophthalate; bis(2,2,6,6-tetramethylpiperidine) terephthalate; or polymeric multifunctional amines such as poly((6-((1,1,3,3-tetramethylbutyl)amino)-1,3,5-triazine-2,4-diyl)((2,2,6,6-tetramethyl-4-piperidinyl)imino)-1,6-hexanediyl((2,2,6,6-tetramethyl-4-piperidinyl)imino)) (CHIMASSORB® 944, Ciba Specialty Chemicals, D-Lampertheim).

One way of carrying out the invention is that in the first step, the secondary amine of formula (III) is introduced into a reaction vessel containing water. The weight ratio of water to secondary amine is in the range of about 0,1 to 200, preferably about 1 to 50, and more preferably about 2 to 30. It is preferred that the water contains a basic inorganic or organic buffer or inorganic or organic bases. The molar ratio of secondary amine to buffer or base is in the range from about 20 to 0,05, preferably about 10 to 0,1, more preferably about 5 to 0,5.

Preferably, the secondary amine of formula (III) is dissolved in a suitable solvent not miscible with water, in order to form a biphasic medium. Preferred solvents are toluene, xylene or dichloromethane. The solvent to secondary amine weight ratio is in the range from about 0,1 to 30, preferably about 0,5 to 10, and more preferably about 1 to 5.

While stirring vigorously, the oxidizing agent (A) is then slowly added in its pure form to the reaction vessel containing the secondary amine of formula (III). It is also possible to add a solution of the oxidizing agent (A) to the reaction vessel. Suitable solvents used for that purpose should be inert towards the various reagents and should not react during the reaction: they are for example toluene, xylene, dichloromethane. When the oxidizing agent (A) is water-soluble, the preferred solvent is water. The solvent to oxidizing agent weight ratio is in the range from about 0,1 to 30, preferably about 0,5 to 10, and more preferably about 1 to 5.

The temperature of the reaction may range from about -10°C to about 130°C, preferably about 0°C to 80°C, and more preferably about 0°C to 50°C. The reaction time may range from about 10 minutes to about 72 h, preferably about 1h to 36 h and more preferably about 2 h to 24 h. The first step of the process of the present invention can be carried out in air or in an inert gas atmosphere such as nitrogen or argon. The pH of the reaction solution can optionally be adjusted to a range from 5 to 8 with substances such as NaHCO₃ for example.

In the second step, after the partial or complete oxidation of the secondary amine to form a nitroxyl radical, stirring is terminated and the aqueous phase is removed.

In the third step, the free radical initiator is added to the organic phase of step two. It is also possible to add a solution of the free radical initiator to the reaction vessel. Suitable solvents used for that purpose should be inert towards the various reagents and should not react during the reaction: they are for example toluene, xylenes, dichloromethane, tetrahydrofurane, dimethylformamide, carbonyl compounds, preferably methyl ethyl ketone or any desired mixtures of the solvents mentioned. The solvent to free radical initiator weight ratio is in the range from about 0,1 to 50, preferably about 0,5 to 25, and more preferably about 1 to 1.5.

The free radical initiator is used in a 0,01- to 10-fold molar excess based on the initial secondary amine. Preferably in a 0,1- to 5-fold molar excess, and most preferably in a 0,25 to 2-fold molar excess based on the initial secondary amine.

The reaction temperature may range from about -10°C to 150°C, preferably 0°C to 100°C, and more preferably 25°C to 85°C. The reaction is most preferably carried out in an inert gas atmosphere, such as in nitrogen or argon.

Preferably, the reaction is carried out until all the free-radical initiator has been decomposed, in order to avoid any contamination of the final product by unreacted free-radical initiator. In the case that the decomposition of the free-radical initiator is not complete it is possible to increase the time or the temperature. It is also possible to add compounds which are able to decompose the excess of free-radical initiator.

After the reaction is complete, some acidic water (pH≈ 5-2) is added to the organic medium and the organic phase is washed several time with acidic water in order to remove the excess of unreacted secondary amine. Optionally, the organic phase can be washed with basic water (pH ≈7,5-9,5) and/or some reducing agents, in order to remove an excess of oxidizing agent (A). The organic phase is then dried under a drying agent such as Na₂SO₄ or MgSO₄. The elimination of the solvent under vacuum provides the crude alkoxyamines of the formulae (I) or (II).

In case of using an organic solvent for the reaction which is miscible with water, it is preferred to eliminate the organic solvent under vacuum before the acidic water is added. For the extraction of the alkoxyamine, it is preferred to use a non-water miscible organic solvent.

Optionally, the alkoxyamines can be further purified by purification methods like recristallization, chromatography or distillation.

Another object of the present invention is to provide a new process for preparing oligomers, cooligomers, polymers or block or random copolymers, which comprises preparing the functional alkoxyamines of formulae (I) or (II) according to the process of the present invention and adding at least one polymerizable monomer to the unpurified alkoxyamine of formulae (I) or (II) followed by heating.

Useful monoethylenically unsaturated monomers are for example alkyl esters of acrylic or methacrylic acids, such as methyl acrylate, ethyl acrylate, butyl acrylate, methyl methacrylate, ethyl methacrylate, butyl methacrylate and isobutyl methacrylate, the hydroxyalkyl esters of acrylic or methacrylic acids, such as hydroxyethyl acrylate, hydroxypropyl acrylate, hydroxyethyl methacrylate and hydroxypropyl methacrylate; acrylamide, methacrylamide, N-tertiary butylacrylamide, N-methylacrylamide, N,N-dimethylacrylamide; acrylonitrile, methacrylonitrile, allyl alcohol, dimethylaminoethyl acrylate, dimethylaminoethyl methacrylate, phosphoethyl methacrylate, N-vinylpyrrolidone, N-vinylformamide, N-vinylimidazole, vinyl acetate, conjugated dienes such as butadiene or isoprene, styrene, styrenesulfonic acid salts, vinylsulfonic acid salts and 2-acrylamido-2-methyipropane-sulfonic acid salts and acryloyl.

Suitable comonomers are C₃-C₆-ethylenically unsaturated monocarboxylic acids as well as the alkali metal salts and ammonium salts' thereof. The C₃-C₆-ethylenically unsaturated monocarboxylic acids include acrylic acid, methacrylic acid, crotonic acid, vinylacetic acid and acryloxypropionic acid. Acrylic acid and methacrylic acid are the preferred monoethylenically unsaturated monocarboxylic acid monomers.

Also suitable comonomers are for example C₈-C₁₆-ethylenically unsaturated phenolic compounds are 4-hydroxy styrene, 4-hydroxy, α-methyl styrene, 2,6-ditertbutyl and 4-vinyl phenol.

Another class of carboxylic acid monomers suitable for use as comonomers according to the present invention are C₄-C₆-ethylenically unsaturated dicarboxylic acids and the alkali metal and ammonium salts thereof as well as the anhydrides of cis-dicarboxylic acids. As examples are mentioned maleic acid, maleic anhydride, itaconic acid, mesaconic acid, fumaric acid and citraconic acid, maleic anhydride and itaconic acid. Preferred monoethylenically usaturated dicarboxylic acid monomer(s).

The acid monomers may be used in the form of the corresponding acids, alkali metal salts or ammonium salts.

Preferred monomers are selected from the group consisting of (meth)acrylic acid esters of C₁-C₂₀-alcohols, acrylonitrile, cyanoacrylic acid esters of C₁-C₂₀-alcohols, maleic acid diesters of C₁-C₆-alcohols, maleic anhydride, vinylpyridines, vinyl(alkylpyrroles), vinyloxazoles, vinyloxazolines, vinylthiazoles, vinylimidazoles, vinylpyrimidines, vinyl ketones, styrene or styrene derivatives which contain a C₁-C₆-alkyl radical or halogen in the α-position and contain up to 3 additional substituents on the aromatic ring.

Particularly preferred monomers are methyl acrylate, methyl methacrylate, butyl acrylate, butyl methacrylate, 2-ethylhexyl acrylate, cyclohexyl methacrylate, isobornyl methacrylate, maleic anhydride, styrene and acrylonitrile.

An important advantage of the process according to the present invention is that an additional purification step of the alkoxyamines can be dispensed with.

For the preparation of the (co)polymers according to the present invention, all the components such as monomer(s), crude alkoxyamine of the formulae (I) or (II) are reacted at a temperature ranging from about 0°C to 260°C, preferably about 50°C to 200°C, and more preferably about 70°C to 150°C, for a period of time ranging from about 30 minutes to 72 hours, preferably about 1 hour to 48 hours, more preferably about 2 hours to 24 hours. The polymerization is carried out in an inert gas atmosphere, for example nitrogen or argon.

Optionally, some additives can be added to the polymerization medium before the polymerization or during the polymerization process in order to accelerate the polymerization. Such additives are well-known in the art and are for example camphorsulfonic acid, 2-fluoro-1-methylpyridinium p-toluenesulfonate, acylating compounds such as acetic anhydride (Tetrahedron 1997, 53(45), 15225), glucose, dextrose (Macromolecules 1998, 31, 7559), ascorbic acid (Macromolecules 2001, 34, 6531) or long-life radical initiators as reported in US-A 6,288,186 (column 4, lines 8-24).

The (co)polymers of the present invention may have a number average molecular weight of from 500 to 2·10⁶, preferably from 2000 to 5· 10⁵, more preferably from 2000 to 2,5·10⁵.

The alkoxyamine compound of the formulae (I) or (II) is introduced in a quantity ranging from about 30 wt% to 0,01 wt%, preferably 15 wt% to 0,05 wt% and more preferably 5 wt% to 0,05 wt%, based on the weight of the monomer(s).

Preferably for the preparation of the (co)polymers only small amounts of organic solvents are used. If organic solvents are required, suitable solvents or mixtures of solvents are typically pure alkanes such as hexane, heptane or cycloalkane, hydrocarbones such as benzene, toluene or xylene, halogenated hydrocarbons such as chlorobenzene, esters such as ethyl acetate, propyl, butyl or hexyl acetate, ethers such as diethyl ether, dibutyl ether or ethylene glycol dimethyl ether, alcohols such as methanol, ethanol, ethylene glycol or monomethyl ether or mixtures thereof of them. The solvent to monomer weight ratio is in the range from about 0 to 5, preferably from about 0 to 2.

The type of polymerization used can be bulk, solution, emulsion, dispersion or suspension polymerization and it can be carried out both batchwise and continuously.

The polymers prepared according to this invention show a low polydispersity (M_{w}/Mₙ) which is usually lower than 2 and can be significantly lower.

The number average molecular weight of the polymer chains increases linearly with the monomer conversion, which allows a tailor-made polymer molecular weight to be obtained. Furthermore, the molecular weight of the polymers can be controlled by varying the amount of crude alkoxyamine compared to the amount of monomers. High molecular weight polymers can be formed.

A further advantage of the present invention is that after the removal of the nonpolymerized monomers from the (co)polymers or after reaching a conversion rate of 100%, a second polymerization step can be initiated simply by adding to the polymer synthesized in the first step a portion of fresh vinyl monomer or monomer mixture that can be different from the vinyl monomer or monomer mixture used in the first polymerization step. The polymerization of the vinyl monomer or monomer mixture added in the second step is then initiated by the polymer chains synthesized in the first polymerization step and di-block copolymers can be for example produced if the polymer chains synthesized in the first polymerization step consists of linear chains with one single growing chain end. The molecular weight and polydispersity of each block can be controlled independently during the respective polymerization step. This process can be repeated several times and can then provide multiblock copolymers of controlled molecular weight and molecular weight distribution for each block.

The resulting polymers are usually colorless and they can be used in most cases without any further purification.

The following examples illustrate the invention in more detail.

### Examples

The molecular weight was determined by gel permeation chromatography (GPC), equipped with a Shodex RI 74 differential refractometer. A flow rate of 1 ml/min was used and samples were prepared in THF. Polystyrene standards were used for calibration.

### Example 1

### Synthesis of 2-methyl-2-[(2,2,6,6-tetramethyl-1-piperidinyl)oxy]propanenitrile (1)

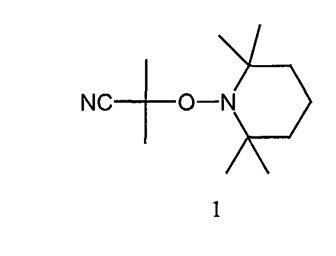

In a 1L four-necked round bottom flask equipped with a mechanical stirrer, a reflux, a thermometer, a funnel and a septum are added 80 g of water, 20 g K₂CO₃ (99 %; 1.44 · 10⁻¹ mol), 20 g 2,2,6,6-tetramethylpiperidine (99 %; 1.41 · 10⁻¹ mol) and 150 g toluene. Then, a solution of 31.3 g of peracetic acid (35 wt%, Aldrich; 1.44 · 10⁻¹ mol) in 300 g water is slowly added to the 1L flask while stirring vigorously (with a slightly exothermic reaction) and the flask is placed in a water bath at room temperature. After the addition, the reaction medium is stirred at room temperature overnight, and the organic phase becomes progressively red due to the formation of 2,2,6,6-tetramethylpiperidine-1-oxide (TEMPO).

Then, the stirring is terminated and the water phase is removed from the reaction flask. The red organic phase is then degassed by bubbling argon for 10 minutes. 11.82 g azobisisobutyronitrile (AIBN, 7.2 · 10⁻² mol) are then slowly added by small portions under an argon atmosphere and while stirring vigorously. Then, 100 g of degassed toluene is added rapidly to the reaction flask and the temperature is increased to 60°C. After 24 h at 60°C, the solution is cooled at room temperature. Finally, this organic solution is washed 3 times with water (pH = 3) and then, dried with Na₂SO₄. After drying under vacuum at 50°C, 17.95 g of crude alkoxyamine **1** is collected.

### Example 2

### Polymerization of styrene initiated by the crude and purified 2-methyl-2-[(2,2,6,6-tetramethyl-1-piperidinyl)oxy]propanenitrile (1) synthesized in example 1.

### 1. Polymerization of styrene using the crude (unpurified) alkoxyamine 1.

To a three-necked round bottom flask equipped with a mechanical stirrer, a reflux condenser, a thermometer and a septum are added 0.242g of unpurified **1** and 100 g of styrene (0.96 mol). The solution is then degassed by bubbling argon for 10 minutes, and is then heated at 125°C.

Samples are picked out the reaction flask after 2.15 and 14 h and dried under vacuum at 70°C during 24h. The yield is calculated by gravimetric analysis and the molecular weights and polydispersity are determined by GPC. The results are shown in table 1.

### 2. Polymerization of styrene using the purified alkoxyamine 1.

The alkoxyamine **1** is purified as follow:

7.31 g of crude alkoxyamine **1** are purified on column chromatography by eluting with a solvent mixture n-hexane/ethylacetate 5/1. 3.68 g of pure alkoxyamine 1 are then collected as a white solid.

1H-NMR: 1.66 ppm (6H): s; 1.51 ppm (6H): m; 1.22 ppm (6H): s; 1.11 ppm (6H): s

### Experimental elemental analysis:

| | | |
|---|---|---|
| C | 69,9%; | 69,8 % |
| H | 10,5 %; | 10,7 % |
| N | 12,4 %; | 12,4 % |

The same procedure as reported for the polymerization of styrene in the presence of the crude alkoxyamine **1** is used, excepted that the crude alkoxyamine **1** is replaced by the purified alkoxyamine **1**. The results are shown in table 1.

**Table 1**

| **Alkoxyamine** | **Time (h)** | **Conversion (%)** | **M**_{**n**} | **M**_{**w**} | **M**_{**w**}**/M**_{**n**} |
|---|---|---|---|---|---|
| Not purified | 2.15 | 9.6 | 7420 | 10300 | 1.38 |
| Not purified | 14 | 81.4 | 41300 | 56110 | 1.35 |
| Purified | 2.15 | 13.4 | 10130 | 12400 | 1.22 |
| Purified | 14 | 80.4 | 36910 | 49700 | 1.34 |

The increase of the molecular weight with the monomer conversion and the low polydispersity are consistent with a controlled polymerization. Moreover, the polymerization is fast (about 80% monomer conversion after 14 h of reaction).

The same polymerization rate and the same molecular weights are observed when using the crude and purified alkoxyamines.

### Example 3

### Polymerization of styrene using the crude alkoxyamine 1 and modification of the molecular weight of the polymer by changing the amount of crude alkoxyamine.

To a three-necked round bottom flask equipped with a mechanical stirrer, a reflux condenser, a thermometer and a septum are added 0.242 g or 0.121 g of crude (unpurified) **1** and 100 g of styrene (0.96 mol). The solution is then degassed by bubbling argon for 10 minutes, and is then heated at 125°C.

After cooling, the polymer is dissolved in chloroform, transferred to an aluminum bag, dried overnight in air and then heated for 24 h at 70°C in vacuo. The yield is calculated by gravimetric analysis.

The results are shown in Table 2.

**Table 2**

| **Crude alkoxyamine/St (wt ratio)** | **Time (h)** | **Conversion (%)** | **M**_{**n**} | **M**_{**w**} | **M**_{**w**}**/M**_{**n**} |
|---|---|---|---|---|---|
| 1/413 | 14 | 81.4 | 41300 | 56110 | 1.35 |
| 1/826 | 14 | 80.0 | 72930 | 123600 | 1.69 |

By changing the amount of crude alkoxyamine **1** in the polymerization medium, the molecular weight of the polymer is accordingly changed, as expected for a controlled polymerization. Moreover, high molecular weight controlled polystyrenes can be synthesized using the crude alkoxyamine.

### Example 4

### Copolymerization of styrene and acrylonitrile initiated by the crude and purified 2-methyl-2-[(2, 2 6, 6-tetramethyl-1-piperidinyl)oxy]propanenitrile (1) synthesized in Example 1.

### 1. Copolymerization of styrene and acrylonitrile using the crude (unpurified) alkoxyamine 1.

To a three-necked round bottom flask equipped with a mechanical stirrer, a reflux condenser, a thermometer and a septum are added 0.242g of crude **1**, 75 g of styrene (0.72 mol) and 25 g of acrylonitrile (0.47 mol). The solution is then degassed by bubbling argon for 10 minutes, and is then heated by reflux.

Samples are picked out the reaction flask after 8 and 24 h and dried under vacuum at 70°C during 24h. The yield is calculated by gravimetric analysis and the molecular weights and polydispersity are determined by GPC. The results are shown in table 3.

### 2. Copolymerization of styrene and acrylonitrile using the purified alkoxyamine 1.

For this experiment, the alkoxyamine **1** is purified as reported in Example 2.

The same polymerization procedure as reported for the copolymerization of styrene and acrylonitrile in the presence of the crude alkoxyamine **1** is used, excepted that the crude alkoxyamine **1** is replaced by the purified alkoxyamine **1**. The results are shown in table 3.

**Table 3**

| **Alkoxyamine** | **Time (h)** | **Conversion (%)** | **M**_{**n**} | **M**_{**w**} | **M**_{**w**}**/M**_{**n**} |
|---|---|---|---|---|---|
| Not purified | 8 | 28.3 | 28330 | 49570 | 1.74 |
| Not purified | 24 | 81.6 | 54150 | 76220 | 1.40 |
| Purified | 8 | 33.8 | 27880 | 40730 | 1.46 |
| Purified | 24 | 78.9 | 47130 | 63500 | 1.34 |

For both cases (crude and pure alkoxyamine), the same rate of polymerization can be observed (about 80% monomer conversion is reached after 24h of polymerization at reflux). Moreover, the polydispersity index is narrow and the molecular weight of the polymer increases with the monomer conversion, in agreement with a controlled process.

### Example 5

### Copolymerization of styrene and acrylonitrile using the crude alkoxyamine 1 and modification of the molecular weight of the polymer by changing the amount of crude alkoxyamine.

To a three-necked round bottom flask equipped with a mechanical stirrer, a reflux condenser, a thermometer and a septum are added 0.242 g or 0.121 g of crude (non-purified) **1**, 75 g of styrene (0.72 mol) and 25 g acrylonitrile (0.24 mol). The solution is then degassed by bubbling argon for 10 minutes, and is then heated at reflux for 24h.

After cooling, the polymer is dissolved in chloroform, transferred to an aluminum bag, dried overnight in air and then heated for 24 h at 70°C in vacuo. The yield is calculated by gravimetric analysis.

The results are shown in table 4.

**Table 4**

| **Crude alkoxyamine/St (wt ratio)** | **Time (h)** | **Conversion (%)** | **M**_{**n**} | M_{w} | **M**_{**w**}**/M**_{**n**} |
|---|---|---|---|---|---|
| 1/413 | 24 | 81.6 | 54150 | 76220 | 1.40 |
| 1/826 | 24 | 69.8 | 89650 | 143800 | 1.60 |

By changing the amount of crude alkoxyamine **1** in the polymerization medium, the molecular weight of the polymer is accordingly changed, as expected for a controlled polymerization. Moreover, high molecular weight controlled poly(styrene-co-acrylo-nitrile) can be synthesized in these conditions.

### Example 6

### Synthesis of poly(styrene-co-acrylonitrile)-b-poly(n-butylacrylate) (SAN-b-PnBuA) block copolymers

To a three-necked round bottom flask equipped with a mechanical stirrer, a reflux condenser, a thermometer and a septum are added 10 g polystyrene-co-acrylonitrile) (SAN) as synthesized using the crude alkoxyamine 1 in example 4, 50 g n-butyl acrylate (0.39 mol) and 3.2 mg ascorbic acid (1.81 10⁻⁵ mol). The solution is then degassed by bubbling argon for 10 minutes, and is then heated at 145°C for 5h. The polymer is then precipitated in methanol and dried under vacuum at 80°C.

**Table 5**

| | **GPC** | | | **GPC-UV** | | |
|---|---|---|---|---|---|---|
| | **M**_{**n**} | **M**_{**w**} | **M**_{**w**}**/M**_{**n**} | **M**_{**n**} | **M**_{**w**} | **M**_{**w**}**/M**_{**n**} |
| SAN first block | 54150 | 76220 | 1.40 | - | - | - |
| SAN-*b*-PnBuA | 68990 | 117300 | 1.70 | 71690 | 117200 | 1.63 |

The SAN first block has been extended as proved by the increase of the molecular weight of the first block.

### Example 7

### Synthesis of the crude alkoxyamine 2-methyl-2-[(2, 2,6, 6-tetramethyl-4-oxo-1-piperidinyl)oxy]propanenitrile 2

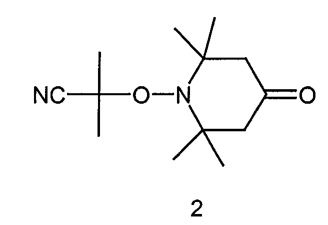

In a 1L four-necked round bottom flask equipped with a mechanical stirrer, a reflux, a thermometer, a funnel and a septum are added 80 g of water, 20 g K₂CO₃ (99 %; 1.44 · 10⁻¹ mol), 22 g 2,2,6,6-tetramethyl-4-piperidone (95 %; 1.41 · 10⁻¹ mol) and 150 g toluene. Then, a solution of 31.3 g of peracetic acid (35 wt.%, Aldrich; 1.44 · 10⁻¹ mol) in 300 g water is slowly added to the 1L flask while stirring vigorously (with a slightly exothermic reaction) and the flask is placed in a water bath at room temperature. After the addition, the reaction medium is stirred at room temperature overnight.

Then, the stirring is terminated and the water phase is removed from the reaction flask. The organic phase is then degassed by bubbling through argon for 10 minutes. 11.82 g azobisisobutyronitrile (AIBN, 7.2 · 10⁻² mol) are then slowly added by small portions under an argon atmosphere and while stirring vigorously. Then, 100 g of degassed toluene is added rapidly to the reaction flask and the temperature is increased to 65°C. After 23 h at 65°C, the solution is cooled at room temperature. Finally, this organic solution is washed 3 times with water (pH = 3) and then, dried with Na₂SO₄. After drying under vacuum at 50°C, 19.68 g of crude alkoxyamine **2** is collected.

### Example 8

### Polymerization of styrene in the presence of the crude alkoxyamine 2.

To a three-necked round bottom flask equipped with a mechanical stirrer, a reflux condenser, a thermometer and a septum are added 0.257 g of crude (unpurified) **2**, and 100 g of styrene (0.96 mol). The solution is then degassed by bubbling argon for 10 minutes, and is then heated at 125°C for 24 h.

Samples are picked out the reaction flask after 2 and 14 h and dried under vacuum at 70°C during 24h. The yield is calculated by gravimetric analysis and the molecular weights and polydispersity are determined by GPC. The results are shown in table 6.

**Table 6**

| **Monomer** | **Time (h)** | **Conversion (%)** | **M**_{**n**} | **M**_{**w**} | **M**_{**w**}**/M**_{**n**} |
|---|---|---|---|---|---|
| **Sty** | 2 | 43.2 | 31240 | 42980 | 1.37 |
| **Sty** | 14 | 92.5 | 50040 | 74330 | 1.48 |

The increase of the molecular weight with the monomer conversion and the low polydispersity are consistent with a controlled polymerization. Moreover, the polymerization is fast (92% monomer conversion after 14 h of reaction).

## Claims

1. One-pot process for the preparation of alkoxyamines of the formulae (I) or (II) 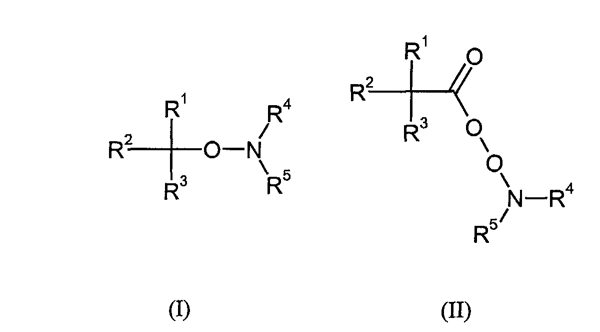 wherein
R¹, R², R³ are independently selected from the group consisting of: hydrogen, C₁-C₂₀alkyl, C₁-C₂₀cycloalkyl, C₆-C₂₄aryl, halogen, cyano, hydroxy, amino, C₁-C₂₀alkylester C₁-C₂₀cycloalkylester, C₁-C₂₀alkylamide, C₁-C₂₀cycloalkylamide C₆-C₂₄-arylester or C₆-C₂₄-arylamide; and
R¹, R², R³ optionally form, together with the intermediate carbon atom, a C₃-C₁₂cycloalkyl radical, a (C₄-C₁₂alkanol)yl radical or a C₂-C₁₃-heterocycloalkyl radical containing oxygen, sulfur or nitrogen atoms;
wherein optionally at least one of the radicals R¹, R² and R³ contains a functional group Y which is capable of further reacting or crosslinking with the functional groups known from the coatings field;
R⁴ and R⁵ are independently selected from the group consisting of: C₁-C₁₈alkyl, C₂-C₁₈alkenyl, C₂-C₁₈alkynyl, C₃-C₁₂cycloalkyl or C₃-C₁₂heterocycloalkyl, C₆-C₂₄-aryl, which are unsubstituted or substituted by NO₂, halogen, amino, hydroxy, cyano, carboxy, ketone, C₁-C₄alkoxy, C₁-C₄alkylthio, C₁-C₄alkylamino, dialkyl phosphonates;
R⁴ and R⁵ optionally form, together with the intermediate nitrogen atom, a C₃-C₁₂cycloalkyl radical, a (C₄-C₁₂alkanol)yl radical or a C₂-C₁₃-heterocycloalkyl radical containing oxygen, sulfur or nitrogen atoms;
R⁴ and R⁵ together form a residue of a polycyclic ring system or a polycyclic heterocycloaliphatic ring system containing oxygen, sulfur or nitrogen atoms;
wherein the carbon atom of the R⁴ and R⁵ radicals directly adjacent to the alkoxyamine nitrogen atom is in each case substituted by 3 further organic substituents;
wherein optionally at least one of the radicals R⁴ and R⁵ contains a functional group Y which is capable of further reacting or crosslinking with the functional groups known from the coatings field;
comprising the reaction steps of
(1) reacting of an oxidizing agent (A) with a sterically hindered secondary amine of the general formula (III), 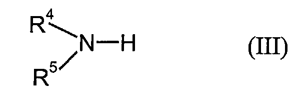 wherein
R⁴ and R⁵ are independently selected from the group consisting of: C₁-C₁₈alkyl, C₂-C₁₈alkenyl, C₂-C₁₈alkynyl, C₃-C₁₂cycloalkyl or C₃-C₁₂heterocycloalkyl, C₆-C₂₄aryl, which are unsubstituted or substituted by NO₂, halogen, amino, hydroxy, cyano, carboxy, ketone, C₁-C₄alkoxy, C₁-C₄alkylthio, C₁-C₄alkylamino, dialkyl phosphonates;
R⁴ and R⁵ optionally form, together with the intermediate nitrogen atom, a C₃-C₁₂cycloalkyl radical, a (C₄-C₁₂alkanol)yl radical or a C₂-C₁₃heterocycloalkyl radical containing oxygen, sulfur or nitrogen atoms;
R⁴ and R⁵ together form a residue of a polycyclic ring system or a polycyclic heterocycloaliphatic ring system containing oxygen, sulfur or nitrogen atoms;
wherein the carbon atom of the R⁴ and R⁵ radicals directly adjacent to the alkoxyamine nitrogen atom is in each case substituted by 3 further organic substituents;
wherein optionally at least one of the radicals R⁴ and R⁵ contains a functional group Y which is capable of further reacting or crosslinking with functional groups known in the coatings field;
in a water-containing medium,
(2) removing of the aqueous phase,
(3) adding a free-radical initiator (B) and starting the decomposition to generate radicals of the general formulae (IV) or (V): 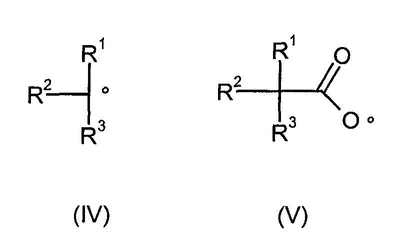
wherein
R¹, R², R³ are independently selected from the group consisting of: hydrogen, hydroxy, cyano, C₁-C₂₀alkyl, C₁-C₂₀cycloalkyl, C₆-C₂₄aryl, halogen, cyano, hydroxy, amino, C₁-C₂₀alkylester C₁-C₂₀cycloalkylester, C₁-C₂₀alkylamide, C₁-C₂₀cycloalkylamide C₆-C₂₄-arylester or C₆-C₂₄-arylamide;
R¹, R², R³ optionally form, together with the intermediate carbon atom, a C₃-C₁₂cycloalkyl radical, a (C₄-C₁₂alkanol)yl radical, a phenyl radical or a C₂-C₁₃-heterocycloalkyl radical containing oxygen, sulfur or nitrogen atoms;
wherein optionally at least one of the radicals R¹, R² and R³ contains a functional group Y which is capable of further reacting or crosslinking with the functional groups known from the coatings field.

2. Process for preparing oligomers, cooligomers, polymers or block or random copolymers, which comprises preparing the functional alkoxyamines of formulae (I) or (II) according to Claim 1 and adding at least one polymerizable monomer to the unpurified alkoxyamine of formulae (I) or (II) followed by heating.
